Europäisches Patentamt

European Patent Office

Office européen des brevets

(19)

(11) Publication number : **0 488 876 A1**

## (12) EUROPEAN PATENT APPLICATION

(21) Application number : 91403185.1

(22) Date of filing : 26.11.91

(51) Int. Cl.⁵ : **A61B 5/0402, A61B 5/0408**

(30) Priority : **26.11.90 ES 9003004**

(43) Date of publication of application :
**03.06.92 Bulletin 92/23**

(84) Designated Contracting States :
**DE FR GB IT**

(71) Applicant : **Fernandez Belmonte, Asensio**
**36, Virgen del Pilar**
**E-08320 El Masnou (ES)**

(72) Inventor : **Fernandez Belmonte, Asensio**
**36, Virgen del Pilar**
**E-08320 El Masnou (ES)**

(74) Representative : **Levy, David et al**
**c/o S.A. FEDIT-LORIOT & AUTRES CONSEILS**
**EN PROPRIETE INDUSTRIELLE 38, Avenue**
**Hoche**
**F-75008 Paris (FR)**

(54) **Electrocardiograph.**

(57) This electrocardiograph comprises electrodes (6,6',7,7') to be disposed on a patient to be examined and means (11,12) to provide electrocardiogram ; it consists in a frame (1) joined to an appropriate chair (3) to accomodate said patient in a normal position ; there are at least four fixed electrodes in the shape of cradle, which are positioned at either side of said chair and at two different heights with respect to the level of a seat (3) of said chair and in which the limbs of the patient are fitted in order to establish a safe electrical contact.

EP 0 488 876 A1

The first electrocardiogram of the human heart was produced over one hundred years ago. Since then, electrocardiography has continued to progress, providing those working in medicine, with tools which are becoming cheaper, lighter, safer and easier to use.

However, there is no electrocardiograh on the market which can be used directly by the patients themselves without the risk of error. There are certainly none available to the general public that do a basic and rapid check on the condition of the heart and which are automatic, functioning in exchange for a predetermined value of coins. Nor is there any such apparatus on the market which can provide physicians or the patient himself with the most relevant data concerning the heart of the person undergoing the examination (blood pressure, weight, height, ect.).

All known electrocardiographs, apart from having been devised solely to carry out an exploratory function, are based on the utilization of electrodes positioned at the extremities of flexible conductors which must be placed on the patient's body in precise positions. These electrodes are fixed in place by suction, plasters, or other methods.

The positioning of these electrodes requires specific knowledge, care, experience and even a certain manual skill. This precludes the possibility of the use of the electrocardiograph by members of the public and even of many physicians who are not specialized in the handling of this type of diagnostic apparatus.

By contrast, the apparatus which is the subject of the present invention, comprises fixed electrodes with which the user will automatically enter in contact when adopting the necessary position. There is no risk of error. Contact between the body of the patient and the electrons happens due to force of gravity, rendering unecessary artificial methods of positioning and manual skills used to place the electrodes. Nor are there risks of defective contacts which could distort results. Moreover, one of the features of this invention is that the apparatus is provided with means of measuring the patient's blood pressure, and at the very least of measuring his height and weight. Thus the patient is provided with a complete picture of his state of health.

It may be seen that this apparatus is particularly useful in the above-mentioned situations (working automatically when a pre-determined value of coins is introduced), although it can also be used to great advantage by medical personnel - particularly by those who are not specialized in the operation of electrocardiographs, for example, general practitioners or interns.

This invention basically consists of a chair, on which the user will be seated in a normal position, and four metal supports which will hold the wrists and ankles of the user whilst he is in a normal resting position. These supports comprise the electrodes of standard unipolar extremes through which the cardiogram is effected, in the known form, giving one of the said supports -to be exact, the one corresponding to the right ankle -the simple function of connecting the body of the user to the earth. Thus, there is no possibility of error, nor any risk of occurrence of defective contacts.

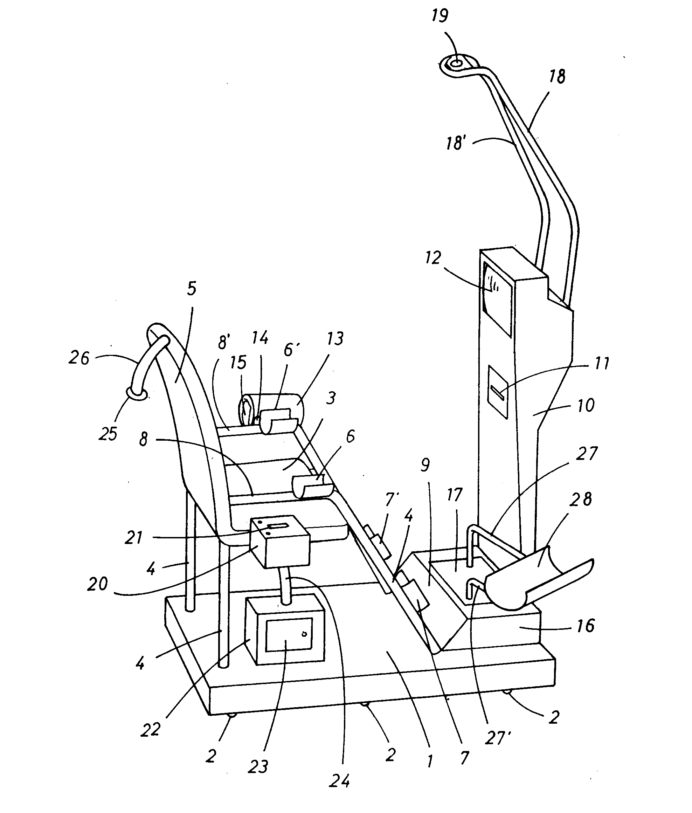

Having given a concrete example of the practical use of this apparatus, the essence of the machine along with its principal characteristics and its advantages will be seen more clearly with reference to the attached drawing.

Preferably, though not necessarily, the apparatus would consist firstly of a base or pedestal (1), with electro insulating characteristics, which rests on the floor - either directly or with a set of supports (2). These supports have the advantage of being adjustable in order to compensate for irregularities or defects of the horizontal which might occur. A chair (3), complete with back (5), is attached to the pedestal by its legs (4). This chair, whose shape and dimensions could of course vary widely, is designed for the user to sit in the normal position. One of the characteristics of this invention is that the seat back (5) is connected to the seat (3), in such a way that it is possible to adjust it to a horizontal or near horizontal position. An adjustable front support has also been planned. The above has been designed with a view to the apparatus being used with the patient lying down, for example, in emergency dispensaries, hospitals, etc. The above-mentioned part of the apparatus must include the metallic structures (6-6') (7-7'), arched and cradle shaped and placed so that the person occupying the chair (3) in the normal position can rest his wrists and ankles. Obstructive clothing would first be removed but may be put on again as soon as the metallic structures are in place. In this way, a perfectly safe and efficient contact is made between these parts (which act as electrodes) and the limbs of the user. Use of conductor creams is not necessary.

It is preferable (but not necessary) that the chair has arms (8) (8') upon which the electrodes (6) (6') are placed to receive the wrists of the user. Preferably, at the front part of the seat there would be an inclined footrest (9) either continuous or not upon which the user might rest his feet and close to which the electrodes (7), (7') would be situated. These electrodes would be attached to the front chair legs (4) and in a position to support the ankles.

The electrodes (6) (6') (7') correspond to the standard unipolar extremes avR, avL and avF and to the D-1, D-II and D-III and the heart extremes V-I, V-II, V-III, V-IV, V-V, V-VI. Electrode (7) serves only to connect the patient to earth, as is normal. However, all of these electrodes could be perfectly well supported by vertical rods fixed to the base (1) or they could be fixed at the appropriate height for any other type of support system. The electrodes 6, 6', 7 and 7' have a rough surface against which the body of the patient will make contact. This surface has the object of increasing the surface contact and of improving the effect of retention.

At the front part of the base (1) there is a vertical column (10) containing the electronic components. These, according to well-known and established technology, effect the user's electrocardiogram, following a pre-established order and analyse both the standard unipolar extremes and the standard bipolar extremes in the normal way. The column also houses the power supply and a printer (also of a well-known type) which, at the end of each examination, issues the printout of the electrocardiogram results through the slot (11) (it will also issue the results of the other data to which reference will later be made), consisting of an interpretation of the data and any anomalies detected. Lastly, at the top of the column (10) there is a screen (12). Again according to well known techniques, the results obtained by the apparatus will appear on the screen along with a basic analysis of these results.

Another feature of the invention is that it incorporates (or would incorporate) means of measuring particular physical characteristics of the user and could check particular physiological data of the same.

To this end, the apparatus has a tubular structure (13) fixed, for example, by supports (14) to one of the arms (8'). This is lined with an inflatable sleeve (15), into which the user can insert his arm end and by which (again, according to proven methods) the blood pressure is measured. Also, the first part of the base/pedestal (1) has an extension (16) separated from the rest of it by an inclined step (9), to which reference has already been made, on which a weighing platform is situated (17) finally, arms (18) (18') emerge from the top of the column (10) and maintain in an elevated position above the vertical of the platform (17), an instrument to measure length by ultrasound (19). This instrument is of a type which is well known and tested and it measures the height of the user when he is on the weighing platform. The data obtained from these instruments (blood pressure, weight and height) are collected and recorded by the printer and are displayed on the screen (12), thus completing the information provided by the apparatus.

With a view to the best method of exploitation or utilization of the apparatus, the following part (20) could be placed in one of several appropriate places on the machine, for example on column (10) or on one of the two sides of the chair (3). The structure (20) comprises the control unit of the apparatus. This basically consists of a coin selector (into which coins would be dropped through a slot (21)), a switch to start the machine and possibly a switch or button allowing one to select functions required. Any other useful elements might be added to this. Along with the coin/slot selector there would naturally be a collection box for the coins (22). This could be fitted, for example, to the base (1) or under the chair (3) with a security door (23). This would obviously be secure and would be connected to the coin selector by an inviolable channel (24) down which the coins would fall into the box.

Although it has been stated that the apparatus would be used preferably by the general public and operated by the insertion of coins, this is by no means the only way of making use of it. On the contrary, it could also be of great help to medical staff. With this is mind, one might want to modify the position of some of the machine's parts, situating, for example, column (10) (with all of the structures it supports) at the back rather than at the front of the chair (3). The structure (20), which supports the control unit, could also be moved to the back. The money selector and related parts would now of course be unecessary. Moreover, it would be possible to equip the apparatus with an exploratory electrode (25) which could then be positioned at the end of a flexible pipe (26) attached, for example, to the top of the seat back (5) and through which it is possible to establish the previous heart extremes and thoracic skin surfaces. The amount of these exploratory electrodes could be increased to a maximum of six, multiplying the exploratory possibilities of the apparatus. Also with regard to the uses we are concerned with, it would also be possible to mount the electrodes (6) (6') (7) (7') to a fixed position, placing them at the ends of flexible support conductors. This would allow them to be applied to any part of the patient's body, adapting them, for example, to a patient with a physical handicap.

The apparatus could also be adapted to use within hospitals, particularly with a view to obtaining continuously check of heart functioning of patients in specific circumstances. With regard to the above, the seating apparatus (an essential part) is adaptable in the sense that one or more of its parts can be adjusted to a horizontal or near horizontal position on which the patient can rest.

Another extremely useful feature of the apparatus is that of the surface texture of the tubes or cradles (6) (6') (7) (7') against which the limbs of the patient will rest. The surface, rather than being smooth, is rough. It would consist, for example, of a succession of small raised vertexes or edges. Apart from aiding immobility of the limbs, this roughness of surface increases their contact area, guaranteeing a perfect electrical contact between surface and limbs, without any of the necessary requisites to achieve this.

Another characteristic of this invention, the presence of the weighing platform (17) can be used to advantage by the addition of baby-scales (28). These would be attached to the above platform by any appropriate set of supports (27) (27') and would complete the possible applications of the invention and would be a particularly useful feature with regard to its installation in chemists and similar establishments.

## Claims

1. An electrocardiograph which comprises electrodes (6,6',7,7') to be disposed on a patient to be examined and means (11,12) to provide electrocardiogram, characterised in that it consists in a frame (1) joined to an appropriate chair (3) to accomodate said patient in a normal position, in that there are at least four fixed electrodes in the shape of cradle, which are positioned at either side of said chair and at two different heights with respect to the level of a seat (3) of said chair and in which the limbs of the patient are fitted in order to establish a safe electrical contact.

2. An electrocardiograph according to claim 1, wherein it further comprises a vertical column (10) joined to the frame (1) and containing said means providing the electrocardiogram, and a screen (12) disposed on said column (10) and on which appear the successive results, interpretation and diagnosis.

3. An electrocardiograph according to one of claims 1 and 2, wherein it further comprises a control unit (20) provided with a slot (21) for the introduction of coins and means such as a switch for determining the commencement of each functional cycle of the machine.

4. An electrocardiograph according to claim 1, wherein the cradle-shaped electrodes (6,6',7,7') have rough surface against which the body of the patient makes contact in order to increasing the surface contact and to improving the effect of retention.

5. An electrocardiograph according to claim 4, wherein the surface of the cradle-shaped electrodes has a succession of vertexes and/or raised edges.

6. An electrocardiograph according to claim 1, wherein the electrodes are provided on arms (8,8') of the chair (3) and are positioned to receive the limbs of the patient and to establish contact with said limbs.

7. An electrocardiograph according to claims 5 and 6, wherein the electrodes receiving the lower limbs of the patient are fixed at an appropriate height to the front legs (4) of the chair (3).

8. An electrocardiograph according to claim 1, wherein it further comprises a tubular structure (13) including an inflatable sleeve (15) for measuring blood pressure of the patient, said tubular structure being fixed at one of the seat sides and at an appropriate height.

9. An electrocardiograph according to claim 1, wherein in addition to said four basic electrodes, it further comprises at least one another electrode (25) mounted onto an end of corresponding flexible support (26), said another electrode being used as exploratory element when disposed on different parts of the body of the patient for examination of the previous heart data.

10. An electrocardiograph according to claim 1, wherein a weighing platform (17) is provided between the front part of the chair and the vertical column, and whose measures are recorded in a printer and appear on the screen.

11. An electrocardiograph according to claim 10, wherein the weighing platform is attached by means of suitable set of supports and a side platform for weighing infants.

12. An electrocardiograph according to claims 1 and 2, wherein it further comprises at least one arm (18) emerging from the top of the vertical column, said arm having at its free end an ultrasound measurer (19) of distances and is situated on the vertical end of the platform, measures the height of the person positioned on the same and the results of these measurements being recorded in the printer and then appear on the screen.

13. An electrocardiograph according to claims 1 and 9, wherein the chair has a back to which are/is attached the flexible support(s) which have the exploratory electrode(s) mounted onto their extremities.

14. An electrocardiograph according to claim 1, wherein the frame adopts the shape of a horizontal platform which can rest directly on the ground and the seat destined to receive the body of the person undergoing examination is securely attached to it.

15. An electrocardiograph according to claim 1, wherein the chair comprises articulated parts which permit it to be transformed into a horizontal or near horizontal surface, ready to receive the body of the person undergoing examination.

European Patent
Office

# EUROPEAN SEARCH REPORT

Application Number

EP    91 40 3185

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y | PROC. OF THE ANN. INT. CONF. OF THE IEEE/EMBS vol. 11, 12 November 1989, SEATTLE, WA. (US) pages 1461 - 1463; T. TOGAWA ET AL.: 'Physiological Monitoring Systems Attached to the Bed and Sanitary Equipments' * page 1462 - page 1463; figure 3 * | 1,6 | A61B5/0402 A61B5/0408 |
| Y | WO-A-9 004 996 (O. FAKHRI) | 1,6 | |
| A | * page 3, line 11 - page 4, line 22 * | 5,7 | |
| A | US-A-4 898 182 (B. HAWKINS ET AL.) * column 2, line 53 - line 66 * * column 4, line 51 - column 5, line 20 * * column 6, line 56 - line 68; figures 1,2 * | 1-3,12 | |
| A | DE-A-3 301 550 (G. BLAUMEISER) | 1,2,8,10 | |
| A | * page 24, line I - line 25; figures 2,3,7,8 * * page 26, line 20 - page 28, line 19 * | 12 | |
| P,A | DE-U-9 103 737 (A. VON SMEKAL) 8 August 1991 * page 5, line 16 - page 8, line 19 * | 1,6,7,15 | TECHNICAL FIELDS SEARCHED (Int. Cl.5 ) |
| | ----- | | A61B |

The present search report has been drawn up for all claims

| Place of search | Date of completion of the search | Examiner |
|---|---|---|
| THE HAGUE | 26 FEBRUARY 1992 | RIEB K.D. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
................................................................
& : member of the same patent family, corresponding
    document

EPO FORM 1503 03.82 (P0401)